# EUROPEAN PATENT APPLICATION

(11) **EP 4 233 926 A2**
(43) Date of publication of application: **30.08.2023**
(21) Application number: 23171103.7
(22) Date of filing: 07.09.2017
(51) Int. Cl.: A61L 27/52

(54) **METHODS OF MANUFACTURING INJECTABLE GELS COMPRISING CROSS-LINKED HYALURONIC ACID AND HYDROXYAPATITE**

(30) Priority: 07.09.2016 US 201662384219 P
(62) Divisional of application: 17848293.1
(71) Applicant: Luminera Derm Ltd., 7120101 Lod (IL)
(72) Inventor: SEGAL, David Dadi, 6436805 Tel Aviv (IL); GOLDBERG, Eran, 5259321 Ramat Gan (IL); SHKLANOVSKY, Lital, 5540101 Kiryat Ono (IL); ZINGER, Ariel, 5248501 Ramat Gan (IL); GOLDSHAID-ZMIRI, Liat, 4976001 Petach Tikva (IL)
(74) Representative: Russell, Tim

(57) **Abstract**

Provided herein are the compositions and methods of manufacturing of injectable gels of cross-linked hyaluronic acid associated to a varying degree with hydroxyapatite.

## Description

### Field of the invention

The present invention relates to composite materials comprising cross-linked hyaluronic acid and hydroxyapatite, methods of manufacturing of such composite materials and use thereof in preparation of cosmetic compositions, of medical compositions, and of pharmaceutical compositions.

### Background

Hyaluronic acid is a common component of cosmetic preparations and is used in several cosmetic procedures, particularly in filling wrinkles. Natural hyaluronic acid has poor in-vivo stability due to rapid enzymatic degradation and hydrolysis and, accordingly, various chemically modified forms of hyaluronic acid (e.g., cross-linked forms, ionically modified forms, esterified forms, etc.) have been prepared to address poor stability.

Hydroxyapatite has a chemical composition which is very similar to that of the mineral phase of bone. Its biological properties and its biocompatibility make it an excellent bone-substitute product. Bone colonization by the substitute is usually highly dependent upon the porous characteristics of the material and in particular on pore size and distribution, and the interconnection between macropores (number and size). The interconnections are tunnels that allow the passage of cells and the circulation of blood between the pores and thus promote bone formation within the substitute. Calcium hydroxyapatite (CaHAp) is a mineral species of the phosphate family, having the formula Ga₅(PO₄)₃(OH), usually written as Ca₁₀(PO₄)₆(OH)₂ to stress the fact that the lattice of the crystalline structure contains two molecules. Hydroxyapatite belongs to the crystallographic apatite family, which are isomorphic compounds having the same hexagonal structure. This compound has been used as a biomaterial for many years in various medical specialties.

Currently, hyaluronic acid or cross-linked versions thereof are used in various gel forms, for example as soft tissue augmentation products, adhesion barriers, and the like.

For example, PCT patent application WO 2013/053457 discloses a composition of two different molecular weight hyaluronic acid polymers cross-linked in a presence of hydroxyapatite. PCT patent application WO 2016/074794 teaches dermal filler compositions in the form of a gel, comprising hyaluronic acid (HA), carboxymethyl cellulose (CMC) and, optionally, microparticles such as calcium hydroxyapatite (CaHAP). PCT patent application WO 2016/025945 teaches a composite material that includes a hyaluronic acid-based gel and a nanostructure disposed within the gel. US patent application publication US 2013096081 provides highly injectable, long-lasting hyaluronic acid-based hydrogel dermal filler compositions made with a di-amine or multiamine crosslinker in the presence of a carbodiimide coupling agent. Korean patent application KR 20110137907 teaches a dermal filler composition that is formed by adsorbing or covalently bonding anionic polymers on the surface of the ceramic beads like hydroxyapatite bead, bioglass bead, calcium carbonate bead, titanium dioxide bead, barium sulfate bead, alumina bead, and zirconia bead. US patent application publication US 2010136070 discloses a cross-linked composition of hyaluronic acid, derivatives of hyaluronic acid or mixtures thereof, alginic acid, derivatives of hyaluronic acid or mixtures thereof and calcium ions. Additionally, US patent applications publication US 20150257989 and US 2015238525 describe cohesive cross-linked hyaluronic acid gel filled with hydroxyapatite particles. US patent application publication US 2011038938 teaches a self-setting injectable composition comprising: cement particles capable of undergoing a cementing reaction when contacted with a suitable setting liquid; and at least one crosslinkable polymer gel, wherein said polymer gel is capable of undergoing ionic crosslinking in the presence of multivalent ions.

There is a need in the art to provide hyaluronic acid composite materials with improved desired properties, such as controlled degradation resistance, either enzymatic or non-enzymatic, and/or spatial swelling behavior, and/or improved rheological properties and/or, mechanical stability, and/or reduced side-effects, and/or biocompatibility, and/or controlled osmoloarity.

### Summary of the invention

Provided are composite materials, methods of manufacturing thereof, and cosmetic, medical or pharmaceutical compositions of hyaluronic acid and hydroxyapatite, as described in further detail below. In one aspect, hyaluronic acid is cross-linked in multiple steps. In another aspect, the hydroxyapatite is added in multiple steps. Optionally, both the hyaluronic acid is being cross-linked in multiple steps, and the hydroxyapatite is being added in multiple steps.

It has been unexpectedly found by the present inventors that cross-linking of hyaluronic acid and addition of hydroxyapatite may be performed in multiple steps, e.g. in at least two steps. According to a method for producing of the composite material of the invention, hydroxyapatite is added to hyaluronic acid in multiple stages, e.g. in two stages, separated by or concomitantly with a cross-linking of the hyaluronic acid, i.e. in a step-wise manner. The resultant composite material includes dispersed, e.g. finely-dispersed, hydroxyapatite particles within the cross-linked gel, split between different functional regions of the cross-linked hyaluronic acid (HA) matrix as defined below, and having a varying degree of association to the HA matrix, such as, for example, tightly associated hydroxyapatite and loosely associated hydroxyapatite, thereby providing a composite material having improved properties. Without being bound by theory, it is believed that as a result of a multi-step process according to the invention, hydroxyapatite particles that are added during different stages of the cross-linking of hyaluronic acid possess different degree of association with the hyaluronic acid matrix, thereby allowing for a gradual release of the hydroxyapatite particles from the matrix.

The composite material may be incorporated into a cosmetic, medical (including surgical), or pharmaceutical preparation. The preparation is usually in a form of a viscoelastic gel. The preparation may comprise hyaluronic acid in concentrations from about 0.2 to 9 % w/w, inclusive. The preparation may further comprise calcium hydroxyapatite in concentrations between from about 5 to 90 %wt. The preparation may further comprise additional material, e.g. drugs, non-limiting examples being local anesthetic, e.g. lidocaine, or hormones, growth factors, steroids.

In a first aspect of the present invention provided herein is a process of manufacturing of a gel product comprising cross-linked hyaluronic acid and hydroxyapatite, said process comprising: combining in an aqueous medium hyaluronic acid or a salt thereof, a cross-linking agent, and conducting a cross-linking reaction in the presence of a first portion of hydroxyapatite; completing the cross-linking reaction; and incorporating a second portion of hydroxyapatite into the so-formed gel.

The cross-linking may be effected by increasing the pH of the medium. The completing of the reaction may be accomplished by allowing the reaction mixture to stand, and/or neutralizing said reaction mixture, e.g. adjusting the pH to about between 6.0 and 7.8, e.g. about 7.

The concentration of hydroxyapatite in the gel may be above 25 weight percent, preferably above 45 weight percent, and further preferably between 50 and 60 weight percent.

The total amount of hydroxyapatite is divided such that the first portion of hydroxyapatite which is present in the cross-linking reaction mixture, is between 5 and 90 weight percent of the total amount of hydroxyapatite. That is, the weight ratio between the first portion and the second portion is between 1:9 and 9:1. Preferably, the first portion of hydroxyapatite is between 5 and 30 weight percent of the total amount of hydroxyapatite. That is, the weight ratio between the first portion and the second portion is preferably between about 1:7 and 1:3, namely, in some embodiments the predominant portion is added in the second portion.

Calcium hydroxyapatite employed in the process may have an average particle size between 25 and 45 micrometers.

The cross-linking agent may be selected from the group consisting of 1,4-butanediol diglycidyl ether, polyethylene glycol) diglycidyl ether, and ethylene glycol diglycidyl ether; preferably 1,4-butanediol diglycidyl ether.

The concentration of said hyaluronic acid in said aqueous medium may be between 0.2 and 9 weight percent, preferably between 0.5-4 weight percent.

The weight ratio between hyaluronic acid and the cross-linking agent in the aqueous medium may be between 15:1 and 2:1.

The process may further comprise introducing additional amount of hyaluronic acid, i.e. free non-cross-linked hyaluronic acid, to the gel after completing the cross-linking reaction.

The resultant gel may be degassed, e.g. in vacuo, filled into a suitable injection device, and sterilized to be suitable for injection to a subject in need thereof.

In a specific embodiment, the process of the invention comprises charging a reaction vessel with water and hyaluronic acid or a salt thereof, stirring to obtain a solution, adding 1,4-butanediol diglycidyl ether, sodium hydroxide and a first portion of calcium hydroxyapatite, maintaining at elevated temperature for a first period and at ambient temperature for a second period, adding phosphate buffer solution and an aqueous acid to achieve a gel with a nearly neutral pH, and incorporating a second portion of calcium hydroxyapatite into the gel, wherein the weight ratio between the first portion of calcium hydroxyapatite and the second portion of calcium hydroxyapatite is between 1:3 and 1:7, and the total concentration of calcium hydroxyapatite is between 50 and 60 weight percent.

In another aspect of the invention, provided herein, is an injectable gel composition comprising cross-linked hyaluronic acid and hydroxyapatite, wherein the concentration of said hydroxyapatite is above 45 weight percent of total weight of the gel, preferably between 50 and 60 weight percent of total weight of the gel.

In a further aspect of the invention provided herein is an injectable gel composition comprising cross-linked hyaluronic acid and hydroxyapatite, wherein the concentration of said hydroxyapatite is above 20 weight percent of total weight of the gel, e.g. above 25 weight percent, more preferably above 45 weight percent, and yet more preferably between 50 and 60 weight percent, and wherein a portion of said hydroxyapatite is inseparable from said gel following centrifugation for 10 minutes under 735 g-force, said portion being at least about fifth of the total amount of hydroxyapatite, e.g. between about a fifth and a third of the total amount of hydroxyapatite, i.e. between 18 and 35 weight percent inseparable from said gel.

In the injectable gel the particles of hydroxyapatite may have an average particle size between 25 and 45 micrometers.

The concentration of said cross-linked hyaluronic acid in the injectable gel composition may be between 0.2 and 9 weight percent.

The structural unit which cross-links the cross-linked hyaluronic acid in the injectable gel corresponds to the cross-linking agent that was used in the process, e.g. 1,4-butanediol diglycidyl ether, poly-(ethylene glycol) diglycidyl ether, or ethylene glycol diglycidylether. The structural unit thus corresponds to the converted forms of the cross-linking agents.

The gel may further comprise non-cross-linked hyaluronic acid.

### Brief descriptions of the drawings

Fig. 1 presents a flowchart of a process for preparing an injectable gel according to an embodiment of the present invention.
Fig. 2 presents small angle X-ray scattering diagram of various gels comprising either only loosely or tightly and loosely associated hydroxyapatite.
Fig. 3 demonstrates a schematic drawing demonstrating an apparatus used for measuring the release of calcium hydroxyapatite from the gels over time.

### Detailed description

Unless the context clearly dictates otherwise, the terms "preparation", "composition", "composite", "formulation" and the like, as used interchangeably herein, should be construed as referring to an injectable gel product of cross-linked hyaluronic acid and hydroxyapatite, as generally described herein.

The terms "tightly associated" and "loosely associated", as used herein in reference to hydroxyapatite in different regions (e.g. "functional regions") of the gel, should be construed as pertaining to regions of the hyaluronic acid gel with different degree of association between hydroxyapatite particles and the cross-linked gel, high and low, respectively. Thus, a material that is referred to as "tightly associated", or "closely associates", should be construed as a region of material wherein hydroxyapatite is being better associated or more densely co-localized with cross-linked HA, per volume unit of the gel. Conversely, "loosely associated" material should be construed as a region of material wherein hydroxyapatite is being less associated or more loosely co-localized with cross-linked HA, per volume unit of the gel. The terms "region", "functional region", "phase" and the like, as used in reference to the tightly and loosely associated hydroxyapatite regions, should be construed as a fraction of hyaluronic acid gel with varying degree of association or co-localization with the hydroxyapatite. It is believed that tightly associated hydroxyapatite particles are not readily separable from the gel, e.g. by centrifugation. It is also believed that the presence of tightly associated fraction of hydroxyapatite may impede separation of more loosely associated particles from the gel.

In the context of the present invention, the terms "apatite", "hydroxyapatite", "calcium hydroxyapatite" and the like, as used herein, refer to a hydroxyapatite mineral of a general formula Ca₁₀(PO₄)₆(OH)₂, of a suitable quality and purity for use/administration, e.g. injection, in humans.

Sometimes, hydroxyapatite may be substituted by other calcium phosphate minerals. The term "calcium phosphate minerals" refers to a family of minerals containing calcium ions (Ca²⁺) together with orthophosphates (PO₄³⁻), metaphosphates or pyrophosphates (P₂O₇⁴⁻) and occasionally hydrogen or hydroxide ions. Non-limiting examples for calcium phosphate minerals that may be used as an alternative to hydroxyapatite are alpha-tricalcium phosphate and beta-tricalcium phosphate. Other particles of biocompatible material may also be suitable.

In some embodiments, hydroxyapatite may be partially or completely substituted with calcium phosphate minerals, particularly with alpha-tricalcium phosphate and/or beta-tricalcium phosphate, and/or a mixture thereof. In the embodiments, wherein the portion of hydroxyapatite is substituted with calcium phosphate minerals, the portion may vary from about 10 weight percent to about 90 weight percent, e.g. between 10-30, or 30-50, or 50-70, or between 70-90, or between 10-70, or 30-90, or 30-70 weight percent. In these embodiments, the weight portion expressed in weight percent, is percentage of the amount described herein for hydroxyapatite. In some embodiments, hydroxyapatite is completely substituted with calcium phosphate minerals; in these embodiments the amounts of calcium phosphate minerals in the compositions are as described herein for hydroxyapatite.

Hydroxyapatite is embedded in the compositions of the present invention as it may act as a dermal filling material, and may induce collagen synthesis. The inventors have further found that the degree of the association of hydroxyapatite to the HA matrix, e.g. particular amount of the tightly associated hydroxyapatite and loosely associated hydroxyapatite within the HA matrix, may be controlled by the manufacturing process, e.g. by the relative amounts of hydroxyapatite added during various steps of the HA matrix formation process.

Hydroxyapatite may be present in the preparation according to the invention generally in concentrations between 5 and 90 % wt, e.g. between 5 and 70 weight percent, or between 20 and 65 weight percent, preferably between 30 and 60 or between 40 to 70 weight percent of total weight of the gel. In some embodiments, hydroxyapatite in the concentration of between 50 and 60 weight percent. In further embodiments, hydroxyapatite concentration is above 25 weight percent, e.g. above 35, or above 45, or 48, or 51, 53, or between 54 and 57 weight percent.

In some embodiments, a portion of hydroxyapatite is inseparable from the preparation following centrifugation. Generally, the inseparable portion may be determined at either at 2040 g-force for 5 minutes, or at 735 g-force for 10 minutes. The inseparable portion may be at least 18 weight percent of total hydroxyapatite, and may be at least one fifth (e.g. 20 %wt), one fourth (e.g. 25 %wt), e.g. up to about one third (e.g. 35 %wt) of total hydroxyapatite. Centrifugation may be performed as generally known in the art, e.g. using Eppendorf 5415C centrifuge (dimensions: (W x H x D) 21.0 cm x 28.0 cm x 28.5 cm) with gel specimens placed into suitable tubes, e.g. 2-mL Eppendorf tubes. With this centrifuge, 2040 g-force is achieved at 5000 rpm, and 735 g-force is achieved at 3000 rpm.

Hydroxyapatite may be provided in a form of a powder, e.g. a plurality of particles. The average particle size may be less than or equal to 650 µm, preferably less than about 200 µm, further preferably less than about 80 um, and may also be less than about 500 nm. Further preferably about at least 75% of hydroxyapatite particles may be of a size between 25 um and 500 µm, or between 25 um and 300 µm, or between 25 µm and 200 µm, or between 25 µm and 100 µm, preferably between 25 µm and 45 um. Alternatively or additionally, at least 75% of the hydroxyapatite particles may be between 1 µm and 100 µm, or between 5 µm and 45 µm, or between 10 µm and 45 µm. The terms "average particle size", "particle size", "weight average particle size" and the like, as used interchangeably herein in reference to the particles of calcium hydroxyapatite, refer to a weight average of a powder particle size distribution, e.g. of calcium hydroxyapatite; i.e. the average value of particle size in a powder bulk taken by weight proportion of each fraction.

In the context of the present invention, the terms "hyaluronic acid", "HA" or "hyaluronate" refer interchangeably to a linear polysaccharide or to its salt, particularly to a nonsulfated glycosaminoglycan, composed of a repeated disaccharide units, each unit consisting of D-glucoronic acid and D-N-acetylglucosamine, via alternating β-1,4 and β-1,3 glycosidic bonds.

Hyaluronic acid may be depicted by the formula 1 below.

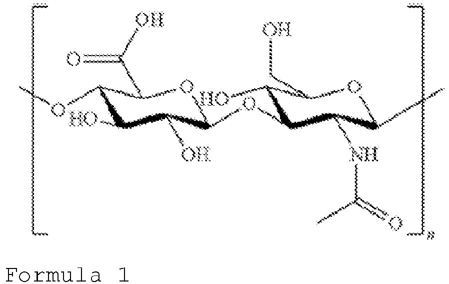

Hyaluronic acid or salts thereof may come from a variety of sources in a variety of molecular weights and other specifications. Generally, all sources of hyaluronic acid may be useful for the purposes of the present invention, including bacterial and avian sources.

The molecular weight of hyaluronic acid may be used as a characteristic to describe the material. The term "molecular weight" includes both the number-average molecular weight, and the weight-average molecular weight, as known for polymers. Useful hyaluronic acid materials may have a molecular weight of from about 0.25 MDa (mega Dalton) to about 4.0 MDa, e.g. from about 0.5 MDa to about 4.0 MDa. Useful ranges of the molecular weight of HA include from about 0.6 MDa to about 2.6 MDa, preferably from about 1.3 MDa to about 2.0 MDa. Useful ranges of the molecular weight of HA may also include from about 1.0 MDa to about 3.0 MDa, from about 1.0 MDa to about 2.5 MDa, from about 1.5 MDa to about 2.0 MDa. Specifically, HA of the molecular weight of about 0.7 MDa, of about 1.8 MDa, or of about 2.7 MDa may be used. In some embodiments, the gel comprises HA having a Mw of between 0.1-5 MDa, between 1 to 5 MDa, between 0.1 to 2 MDa, between 0.1 to 1 MDa, between 1 to 4 MDa, between 2 to 3.5 MDa or between 0.1 to 5 MDa.

Hyaluronic acid may be further characterized with a polydispersity value, indicative of the variation of the molecular weights in the polymer. While it may be advantageous to use a low-polydispersity hyaluronic acid for the sake of improved repeatability of the processes, it may be economically infeasible. A reasonable compromise between the width of the molecular weights polydispersity and the price of the starting material may be achieved, and suitable hyaluronic acid materials may have a polydispersity from about 1.1 to 4.0, preferably less than 3.0, further preferably less than 2.0.

The concentration of cross-linked hyaluronic acid in the composition may vary from 0.2 weight percent to 9 weight percent. In some embodiments, the concentration of hyaluronic acid is between 0.5 and 8, or between 0.8 and 7, or between 0.2 and 1, or between 0.5 and 1.5, or between 0.5 and 4, or between 3 and 6, or between 1 and 5 weight percent. Generally, the cross-linked hyaluronic acid in the composition is hyaluronic acid that was combined with a cross-linking agent at cross-linking conditions, as described herein.

Hyaluronic acid may be at least partially cross-linked. The term "cross-linked" as used herein in reference to hyaluronic acid should be construed as chemical or physical modification of two or more polymer chains of hyaluronic acid, resulting in hyaluronic acid chains being bonded together, preferably covalently bonded. The process of cross-linking may preferably include a cross-linking agent. Similarly, a process of intermolecular or intramolecular reaction without a cross-linking agent, which results in a lactone, an anhydride, an ether, or an ester formation, either within a single polymer chain or between two or more chains. The term "cross-linked" may also be used in reference to hyaluronic acid covalently linked to a cross-linking agent, or to a covalently modified hyaluronic acid.

Additionally, the gel may further comprise free hyaluronic acid, i.e. non-cross-linked hyaluronic acid. Generally, free hyaluronic acid is not exposed to cross-linking conditions. In some embodiments the gels may comprise free hyaluronic acid, and the free hyaluronic acid may be present in the concentrations between 5 to 95 weight percent of total hyaluronic acid. Thus, the ratio of cross-linked to non-cross-linked HA may be at least 0.1:1, e.g. at least 0.5:1, or 1:1, or 2:1, or 5:1, or 10:1.

The term "cross-linking agent" as used herein refers to molecules that contain at least two reactive functional groups that create covalent bonds between two or more molecules of hyaluronic acid. The cross-linking agents can be homo-bifunctional (i.e. have two reactive ends that are identical) or hetero-bifunctional (i.e. have two different reactive ends). The cross-linking agents suitable for use in the present invention usually comprise complementary functional groups to that of hyaluronic acid such that the cross-links could be formed. Preferably, the cross-linking does not form esterified hyaluronic acid. Non-limiting examples of cross-linking agents suitable for the present invention include 1,4-butanediol diglycidyl ether (BDDE), 1,2,7,8-diepoxyoctane (DEO), biscarbodiimide (BCDI), adipic dihydrazide (ADH), bis(sulfosuccinimidyl)-suberate (BS3), hexamethylenediamine (NMDA), 1-(2,3-epoxypropyl)-2,3-epoxycyclohexane, multifunctional cross-linking agents such as pentaerythritol tetraglycidyl ether (PETGE) or PEG based such as polyethylene diglycidyl ether (PEGDE), mono ethylene glycol diglycidyl ether (EGDE), or a combination thereof. Preferably, the cross-linking agent is BDDE.

As used interchangeably herein, the terms "PEG-based cross-linking agent" and the like, refer to polyethylene glycol (PEG) derivatives. The term "PEG" refers to a polyethylene glycol polyether compound with many applications from industrial manufacturing to medicine. PEG is also known as polyethylene oxide (PEO) or polyoxyethylene (POE), depending on its molecular weight. The structure of PEG is commonly expressed as H-(O-CH₂-CH₂)ₙ-OH. Non-limiting examples of PEG derivatives that may be used as cross-linking agents are PEG epoxides, such as poly(ethylene glycol) diglycidyl ether, PEG-dihydrazide, PEG-dihalides, diazide-PEG, diaminooxy-PEG, diamine-PEG, etc.

The general term "cross-linking conditions" as used herein refers to reaction conditions that allow formation of covalent bonds between HA chains. Generally, cross-linking conditions effect the cross-linking reaction, and may include adjustment of the mixture to a desired pH and temperature, specific for a cross-linking agent used. The cross-linking conditions may include adjusting the pH of the mixture to a pH above 12. The cross-linking conditions may further include exposing the mixture to elevated temperature, e.g. to 45°C, for a first period, e.g. between 1 and 5 hours, e.g. 3 hours. The cross-linking conditions may further include exposing the mixture to 25 °C for a second period, e.g. 15 hours. The optimal cross-linking temperature and pH may be readily determined experimentally by testing the cross-linking conditions for HA that are well known in the art for a specific cross-linking agent. Sometimes, the cross-linking conditions may be actively withdrawn, to terminate the cross-linking reaction. The termination of the cross-linking reaction may include adjustment of the mixture to a desired pH and temperature, specific for a cross-linking agent used, e.g. by adjusting the pH of the mixture to a pH of about 7.

The cross-linking degree may be expressed as a percentile of reactive groups of HA that were occupied upon completion of the cross-linking process. The cross-linking degree may be important to the physicochemical properties of the resultant gels, e.g. the degradation rate and/or resistivity to enzymatic degradation. The modification degree (MoD) as defined by the ratio of cross-linker moles to HA dimer moles may be between 1%-40%, 2%-30%, 3%-20%, 4%-10%, particularly 6%-10%.

Cross-linking of HA may be achieved by dissolving/dispersing hyaluronic acid in a solvent, preferably water, adding the cross-linking agent and preferably at least one additive, e.g. hydroxyapatite, and bringing the mixture to cross-linking conditions. Alternatively, the cross-linking agent may be gradually added to a mixture of hyaluronic acid with optional additives, under cross-linking conditions.

The composite gels may further comprise biologically active material, e.g. drugs. The non-limiting examples of drugs suitable for the composite gels include local anesthetic, e.g. lidocaine, and also hormones, growth factors, and steroids.

The composite gels of the present invention may be formed into a pharmaceutical, a medical or a cosmetic preparation. The preparation may usually be an aqueous formulation comprising cross-linked hyaluronic acid or a salt thereof, and calcium hydroxyapatite, preferably in form of an injectable gel, optionally a sterile injectable gel. Further preferably, the preparation is a cosmetic preparation.

In conducting the process according to the invention, hyaluronic acid or a salt thereof may be added to water and mixed in a suitable mixer until dissolution. Cross-liking agent, e.g. BDDE, may be added to the mixer, and mixed until dissolution. Alternatively, a solution of cross-linking agent may be added to the solution of hyaluronic acid. Hydroxyapatite may be dispersed in the reaction mixture, e.g. using a rotor-stator homogenizer.

The conducting a cross-linking reaction may comprise increasing the pH of the medium. This may be achieved by adding to the reaction mixture a sufficient amount of a base or a solution of a base, and mixing until homogeneous. The temperature of the reaction mixture may be elevated if needed. Completing the cross-linking reaction to obtain a gel may include neutralizing said reaction mixture, i.e. to achieve a pH of about between 6.0 and 7.8, e.g. about 7, e.g. by adding an aqueous acid or a neutral or acidic buffer, or allowing the reaction to proceed to an essentially full conversion of the cross-linking agent.

Preferably, the gel formulation comprises:
i) water;
ii) cross-linked hyaluronic acid or one of its salts, at a concentration between 0.2 and 9 %wt (w/w);
iii) hydroxyapatite at a concentration between 5 and 90 %wt (w/w), as described herein, split between a first portion and a second portion; and optionally
iv) drug, for example a local anesthetic, such as lidocaine, up to 1 % w/w.

The formulation may further comprise buffering agents and osmolarity agents, e.g. sodium chloride, phosphate salts, and the like. Phosphate salts may include monobasic, dibasic or tribasic salts of ortho-phosphoric acid with sodium and/or potassium.

The concentration of hyaluronic acid in the gel formulation may range from 0.2 to 9 %wt, or from 1 to 6 %wt, or from 1.5 to 5 %wt, or from 2.5 to 4.5 wt%, or about 4 %wt. In some embodiments, the concentration of hyaluronic acid is between 0.5 and 8, or between 0.8 and 7, or between 0.5 and 1.5, or between 0.5 and 1, or between 3 and 6, or between 1 and 5 weight percent. Preferably, hyaluronic acid concentration is between 0.5 and 4 % w/w.

According to one embodiment, calcium hydroxyapatite particles are present in the formulation at a concentration of between 55 and 57 weight percent, e.g. 55.7% w/w.

The hydroxyapatite particles may be contained in the formulation in various regions with varying degree of association to the HA matrix, e.g. as tightly associated hydroxyapatite, and as loosely associated hydroxyapatite, according to the process of manufacture thereof. The tightly associated hydroxyapatite may be obtained by e.g. adding a portion of hydroxyapatite to a solution comprising cross-linkable hyaluronic acid, e.g. free hyaluronic acid, or a partially cross-linked hyaluronic acid, adding a cross-linking agent, and exposing the mixture to cross-linking conditions. The loosely associated hydroxyapatite may be obtained by e.g. introducing hydroxyapatite to a cross-linked hyaluronic acid, and preferably subjecting the mixture to homogenization. The total amount of hydroxyapatite may thus be split into a first portion and a second portion.

The suitable amount for the first portion of hydroxyapatite (e.g. "tightly associated hydroxyapatite") may be between 1 and 20, preferably between 1 and 10 %wt of the total weight of the finished product (after summing up all the ingredients). The first portion may be between 5 and 90 weight percent of total amount of hydroxyapatite. In some embodiments, the first portion may comprise between 10 and 70 weight percent, between 20 and 60 weight percent, between 30 and 50 weight percent.

The suitable amount for the second portion of hydroxyapatite (e.g. "loosely associated hydroxyapatite") may be from 5-90 weight percent of total amount of hydroxyapatite. In some embodiments, the second portion may comprise between 10 and 70 weight percent, between 20 and 60 weight percent, between 30 and 50 weight percent.

In exemplary embodiments, wherein the composition comprises 30 %wt of hydroxyapatite, the first and second portions may have respective ratio 5:25, or 10:20, or 15:15, or 20:10. In further exemplary embodiments, wherein the composition comprises 40 %wt of hydroxyapatite, the first and second portions may have respective ratio 5:35, or 10:30, or 15:25, or 20:20. In further exemplary embodiments, wherein the composition comprises 55.7 %wt of hydroxyapatite, the first and second portions may have respective ratio 10:45.7, or 20:35.7. In further exemplary embodiments, wherein the composition comprises 70 %wt of hydroxyapatite, the first and second portions may have respective ratio 5:65, or 10:60, or 15:55, or 20:50.

The process of the invention may be conducted by the following steps: a) preparation of a first mixture comprising water and at least 0.2% to 9° weight of hyaluronic acid or a salt thereof, and a cross-linking agent; b) exposing the mixture to cross-linking conditions; c) addition of hydroxyapatite at a concentration between 1 %wt to 20 %wt and exposing the mixture to cross-linking conditions, d) neutralizing the mixture, e) addition of hydroxyapatite at a concentration between 5 %wt to 70 %wt and homogeneously dispersing it in the cross-linked hyaluronic acid gel. Additionally or alternatively, the first part of the hydroxyapatite may be added to the reaction mixture in step a). Additionally, the neutralization step may be performed prior to addition of a consecutive portion of hydroxyapatite. Moreover, the method may further comprise addition of an additional portion of same or different cross-linking agent, and subsequent subjecting the mixture to cross-linking conditions. Thus, more than one neutralization step may be present, as described below.

The pH and osmolarity of the aqueous formulation may further be adjusted to physiological values. Neutralization may be carried out by addition of aqueous solutions comprising pharmaceutically acceptable acids, buffering agents, e.g. phosphate salts, of pH between 6 and 8, according to the requirement of the final pH.

Similarly, osmolarity adjustment may be performed by adding to the mixture a solution of salts, e.g. sodium chloride, phosphates as described herein, and mixing the formulation to obtain homogeneous gel.

At any step of the manufacturing process, the mixture may be tested for quality assurance purposes. The applicable standard tests are known to a technically skilled person and include e.g. rheometry, pH determination, residual cross-linking agent quantification, microscopy, sedimentation by centrifugation, and others.

The ready formulation may be milled, e.g. by extrusion, or by a high-shear mixer, to improve the flow properties prior to packaging. The milling may be performed in presence of additional liquid constituents, e.g. water and/or neutralization and/or osmolarity adjustment solution.

The gels may be readily injectable. In some embodiments, the gels are injectable through a regular medical or cosmetic needle, e.g. 25G/16-mm needle. The term "injectable" should be construed than no excessive force is required to inject the gel through a needle at common injection rate. The injection rate may be from 0.2 mL per minute to 1.5 mL per minute, preferably between 0.9 mL/min and 1.1 mL/min. The force required to inject the gels may vary according to their respective composition and the concentration of hydroxyapatite and hyaluronic acid, but generally when extruded through the 25G needle the average force required to force the gel from a standard 1-mL syringe with 6.35 ± 0.1 mm inner diameter is less than 40 Newton.

The formulation may be filled into syringes and sterilized, e.g. by autoclaving, or by gamma irradiation.

The sterile formulation may be used in a variety of applications, e.g. in tissue filling, such as wrinkle filling or bone graft filling.

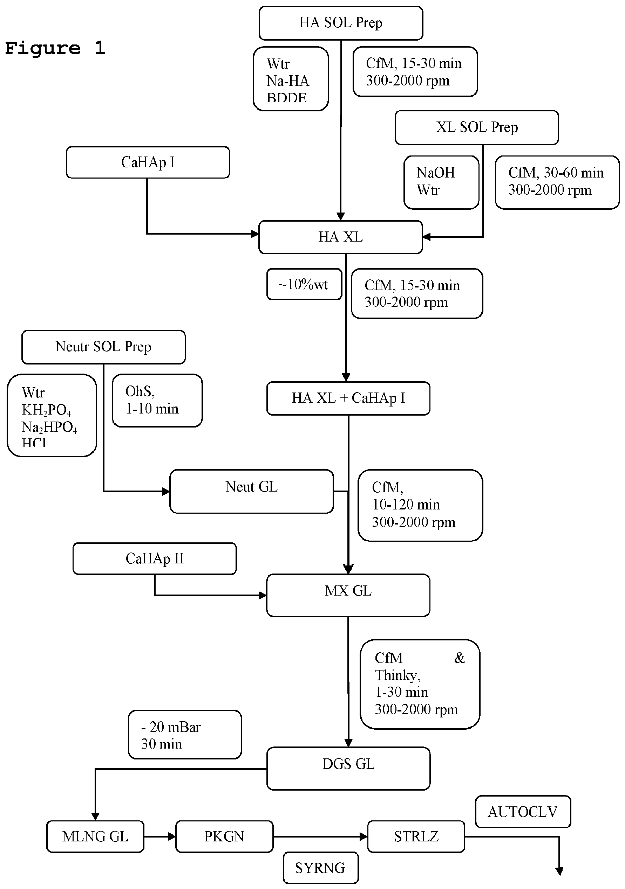

Fig. 1 presents an exemplary flowchart of a process according to an embodiment of the present invention. Preparation of hyaluronic acid solution, termed as "HA SOL Prep", is carried out by dissolving sodium hyaluronate ("Na-HA") and butanediol diglycidyl ether ("BDDE") in water ("Wtr"), in a centrifugal mixer ("CfM"), for 15-30 minutes, at 300-2000 rpm. The cross-linking solution preparation, termed as ("XL SOL Prep"), is carried out by mixing sodium hydroxide in water ("Wtr"), in a centrifugal mixer ("CfM"), for 30-60 minutes, at 300-2000 rpm. The hyaluronic acid cross-linking ("HA XL") is performed by combining HA solution, cross-linking solution and a first portion of calcium hydroxyapatite ("CaHAp I"), about 10 %wt, in a centrifugal mixer ("CfM"), for 15-30 minutes, at 300-2000 rpm. The neutralization solution ("Neutr SOL prep") is prepared by mixing water ("Wtr"), potassium dihydrogen phosphate ("KH₂PO₄"), di-sodium hydrogen phosphate ("Na₂HPO₄"), and hydrochloric acid ("HCl"), in overhead stirrer ("OhS") for 1-10 minutes. The mixture of hydroxyapatite with cross-linked hyaluronic acid ("HA XL + CaHAp I") is mixed with neutralizing solution in a centrifugal mixer ("CfM"), for 10-120 minutes, at 300-2000 rpm, to furnish neutralized gel ("Neut GL"). Additional hydroxyapatite ("CaHAp II") is added to the gel and mixed ("MX GL"), in a centrifugal mixer ("CfM") and/or planetary mixer ("Thinky") for 1-30 minutes, at 300-2000 rpm. The gel is degassed ("DGS GL") at about 20 mBar (absolute pressure) for 30 minutes, then milled ("MLNG"), packaged ("PKGN") in syringes ("SYRNG"), and then sterilized ("STRLZ") by autoclaving ("AUTOCLV").

### Examples

### Example 1 -

### Preparation of gel #1- gel with tightly and loosely associated hydroxyapatite

### Step 1: Preparation of a cross-linked gel based on hyaluronic acid and calcium hydroxyapatite.

Sodium hyaluronate (HA) of molecular weight 1.3-2.0 MDa (pharma grade), 2.31 g, was added to 19.19 g water, mixed to dissolution at room temperature, at 300 rpm using a centrifugal mixer, manufactured by Collomix, followed by 0.23 g of 1,4-butanediol diglycidyl ether (BDDE) (supplied by TCI), and the mixture was further mixed for 30 min at 300 rpm. Thereafter, 3.98 g of 1M sodium hydroxide (NaOH) solution was added to the mixture, bringing to a total weight of 25.71 g, at pH >12. The mixture was then homogenized for 60 min at 300 rpm using the centrifugal mixer. Then, calcium hydroxyapatite Ca₁₀(PO₄)₆(OH)₂ dense microspheres (medical grade), with an average particle size of 25-45 micrometers, 24.00 g, was added to the mixture, bringing to a total weight of 49.71 g, and then mixed again for 30 min at 300 rpm. The mixture was then placed in an oven set to 45 °C for 3 hours, and then placed at 25 °C for additional 15 hours.

### Step 2: Preparation of the final bulk.

The mixture was neutralized by adding 80.61 g of neutralization solution to pH 7. The composition of the neutralization solution was 0.56 g of potassium dihydrogen phosphate (KH₂PO₄) (Pharma grade, supplied by Merck), 0.56 g of disodium hydrogen phosphate (Na₂HPO₄) (Pharma grade, supplied by Merck), 76.66 g of water for injection, and 2.83 g of 1M hydrochloric acid (HCl) solution (Pharma grade, supplied by Merck). The mixture was mixed for 1 hour at 300 rpm using the centrifugal mixer, until a homogeneous gel was formed. A second portion of calcium hydroxyapatite, 109.68 g, was added to the 130.32 g of gel formed after neutralization, and the mixture was further homogenized for 30 min at 300 rpm. The gel was finally degassed in *vacuo,* by subjecting it to 20 mbar vacuum for 30 minutes, filled into 1.25 mL glass syringes, and sterilized in a steam autoclave at 121 °C for 20 minutes. A cohesive and viscoelastic gel was formed.

The hyaluronic acid concentration in the gel (not including the embedded calcium hydroxyapatite microspheres) was 20 mg/g (2% w/w), its pH was ~7.0 and its osmolality was ~300 mOsm/kg. The gel had a final 55.7% concentration of calcium hydroxyapatite, 10% as tightly associated hydroxyapatite and 45.7% as loosely associated hydroxyapatite.

### Test 1 - injectability determination

The extrusion force (EF) was measured by determining the maximum force required to inject the gel thought a 25G/16 mm needle with inner diameter of 0.31 mm (manufactured by PIC, cat no. 03.070250.300.800). Briefly, the gel were inserted to 1-mL BD syringes with inner diameter of 6.35 mm (±0.1), and extruded via the needle at a rate of 1 mL/min. The force was measured by extrusion force measuring device (prepared in-house), which used YISIDA DS-2 force gauge, and the force gauge software (ShanheDS2 Eng). Average extrusion force measured was 34 Newton.

### Example 2 - comparative, preparation of gel #2, with only loosely associated hydroxyapatite

### Step 1: Preparation of a cross-linked gel.

Sodium hyaluronate (HA) of molecular weight 1.3-2.0 MDa, 2.31 g, was added to 19.19 g water, mixed to dissolution at room temperature, at 300 rpm using a centrifugal mixer, manufactured by Collomix, followed by 0.23 g of 1,4-butanediol diglycidyl ether (BDDE) (supplied by TCI), and the mixture was further mixed for 30 min at 300 rpm. Thereafter, 3.98 g of 1M sodium hydroxide (NaOH) solution was added to the mixture, bringing to a total weight of 25.71 g, at pH >12. The mixture was then homogenized for 90 min at 300 rpm using the centrifugal mixer. The mixture was then placed in an oven set to 45 °C for 3 hours, and then placed at 25 °C for additional 15 hours.

### Step 2: Preparation of the final bulk.

The mixture was neutralized by adding 80.61 g of neutralization solution to pH 7. The composition of the neutralization solution was 0.56 g of potassium dihydrogen phosphate (KH₂PO₄) (Pharma grade, supplied by Merck), 0.56 g of disodium hydrogen phosphate (Na₂HPO₄) (Pharma grade, supplied by Merck), 76.66 g of water for injection, and 2.83 g of 1M hydrochloric acid (HCl) solution (Pharma grade, supplied by Merck). The mixture was mixed for 60 min at 300 rpm, until a homogeneous gel was formed. Calcium hydroxyapatite Ca₁₀(PO₄)₆(OH)₂ dense microspheres (medical grade), with an average particle size of 25-45 micrometers, 133.68 g, was added to the 106.32 g of gel formed after neutralization, and the mixture was further homogenized for 60 min at 300. The gel was finally degassed *in vacuo,* by subjecting it to 20 mbar vacuum for 30 minutes, filled into 1.25 mL glass syringes, and sterilized in a steam autoclave at 121 °C for 20 minutes. A cohesive and viscoelastic gel was formed.

The gel had a final 55.7% concentration of calcium hydroxyapatite, as loosely associated hydroxyapatite.

### Example 3 - testing the release from the gels

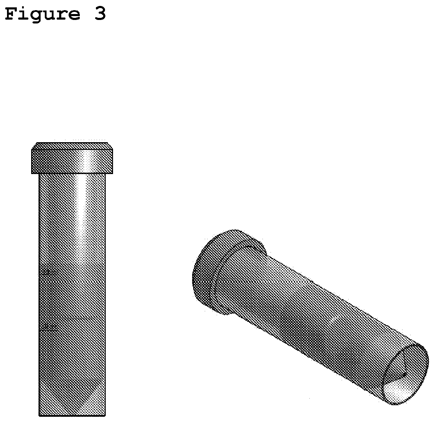

Aliquots of about 1 g of the gels 1 and 2 of Example 1 and of Preparation 1, respectively, were placed into metal mesh inserts (pockets) of total area 81 cm², with wires of ca 40 um and pores of ca 50 um. The inserts were placed into standard 50-mL centrifuge tubes, and 30 mL of water were added into each tube such that in which about 28.5 cm² of the metal pocket was immersed in water. The samples were left for predetermined time intervals as detailed in the Table 1 below, each sample for a specified interval. During the experiment, the gels absorbed water and gradually released calcium hydroxyapatite particles to the water. A schematic representation of the system is demonstrated in the Figure 3.

The content of the calcium hydroxyapatite particles released to the water in each tube was tested and quantified gravimetrically. In brief, the metal pocket was removed and the tube was centrifuged. Then, most of the water was removed by decantation. The sediment of calcium hydroxyapatite particles was washed with about 7 ml of ethanol and dried at 70 °C overnight. The results are summarized in the Table 1.

**Table 1**

| **Duration (days)** | **Gel** | **Gel weight (gr)** | **Dry CaHAp weight (gr)** | **CaHAp, % of the gel** |
|---|---|---|---|---|
| 1 | Gel #1 | 1.0173 | 0.1402 | 13.78 |
| | Gel #2 | 1.0557 | 0.2874 | 27.22 |
| 6 | Gel #1 | 1.0908 | 0.3588 | 32.90 |
| | Gel #2 | 1.0355 | 0.4323 | 41.75 |
| 17 | Gel #1 | 1.0383 | 0.3787 | 36.47 |
| | Gel #2 | 1.0487 | 0.4312 | 41.11 |

It can be readily seen that less calcium hydroxyapatite particles were released from gel #1 in comparison to gel #2 at each time point, i.e. the release kinetics from gel#1 is slower than from the gel#2. This is due to calcium hydroxyapatite being tightly associated with the cross-linked gel in gel #1, unlike the particles in the gel #2, which were only loosely associated with the gel.

### Example 4 - Strength of association of hydroxyapatite

The gels were tested by placing accurately weighed aliquots of about 1.25 g into 2-mL Eppendorf tubes and centrifuged at 2040 g-force, using Eppendorf 5415C centrifuge (dimensions: (W x H x D) 21.0 cm x 28.0 cm x 28.5 cm) at 5000 rpm, for 5 minutes, or at 735 g-force, at 3000 rpm for 10 minutes.

Following centrifugation, two phases were observed, the lower phase contained the calcium hydroxyapatite particles that were separated from the gel and the upper phase contained the remaining gel moiety with particles that were still attached to the gel. The two phases were separated. The gel in the upper phase was digested using hyaluronidase, calcium hydroxyapatite particles were pelletted by centrifugation, washed with water and dried and quantified as described in the Example 3. Calcium hydroxyapatite particles in the lower phase were also dried and quantified as in the Example 3.

As the gel before centrifugation was weighed, the percentage of CaHAp in each phase could be calculated. The percentage of calcium hydroxyapatite was calculated by using the formula: (dry CaHAp weight in each phase)/ (total amount of gel) X 100°.

The results, for 735-g centrifugation, are presented in Table 2 below. Minor deviations from the theoretical 55.7 % calcium hydroxyapatite loading might be attributed to weighing errors.

**Table 2**

| **Gel** | | **Total Gel weight (gr)** | **Dry CaHAp weight (gr)** | **% CaHAp of total gel** | **% CaHAp - Total** |
|---|---|---|---|---|---|
| **Test 1** | Gel #1 (gel) | 2.0308 | 0.2370 | 11.67 | 55.07 |
| | Gel #1 (sediment) | | 0.8813 | 43.40 | |
| **Test 2** | Gel #2 (gel) | 1.8131 | 0.0599 | 3.30 | 50.38 |
| | Gel #2 (sediment) | | 0.8535 | 47.07 | |

### Example 5 - Comparison between a prior art gel (loosely associated hydroxyapatite only) and a gel with both tightly and loosely associated hydroxyapatite

Two further gels were prepared and their characteristics were compared. The first gel (gel#3) was prepared as described in US patent application published as US20150257989, in which the CaHAp is loosely associated with the cross-linked gel. The second gel (gel#4) was prepared according to the same procedure; however, calcium hydroxyapatite was added in two discrete steps as described in the present application, to create tightly and loosely associated CaHAp in the final gel. The final concentration of calcium hydroxyapatite in both gels was 30 weight percent.

In brief, gel#3 was prepared as follows:
Step 1: 3.75 g of sodium hyaluronate of molecular weight 1.3-2.0 MDa, was added to 30.5 g of 1.0% (0.25 M) NaOH. The mixture was left to homogenize for 90 min. Then, 420 mg of 1,4-butanediol diglycidyl ether (BDDE) were added to the mixture, which was homogenized for 5 minutes, closed and placed in an oven at 50 °C for 2 hours. The procedure was then adapted by retaining the mixture for additional 15 h at 25 °C to obtain workable gel. The mixture was thereafter neutralized by adding 7.5 g of HCl 1N.

The gel was purified for 24 h by dialysis with a phosphate buffer (KH₂PO₄ : Na₂HPO₄, ratio 1:1) to obtain final hyaluronic acid concentration of 25 mg/ml (2.5%) and then it was homogenized for 90 min. Gel weight after dialysis was 139.29 g.

Step 2: 100 g of the prepared gel were taken and mixed with 42.9 g of CaHAp (particle size - 25 ~ 45 um) for 90 min until homogenization. The homogenized gel was degassed, filled into 1.25 ml syringes and sterilized by steam autoclave at 130 °C for 3 min.

### Gel #4 was prepared according to the following procedure:

Step 1: 3.75 g of sodium hyaluronate of molecular weight 1.3-2.0 MDa, was added to 30.5 g of 1.0% (0.25 M) NaOH. The mixture was left to homogenize for 90 min. Then 420 mg of 1,4-butanediol diglycidyl ether (BDDE) were added and mixed for 5 min. Thereafter the first portion of calcium hydroxyapatite, 19.90 g (particle size - 25 ~ 45 um) were added to the mixture and homogenized for 30 min, closed and placed in an oven at 50 °C for 2 hours, followed by additional 15 h at 25 °C. The mixture was then neutralized by adding 7.5 g of HCl 1N.

The gel was purified for 24 h by dialysis with a phosphate buffer (KH₂PO₄: Na₂HPO₄, ratio 1:1) to obtain final hyaluronic acid concentration of 25 mg/ml (2.5%) and then was homogenized for 90 min. Gel weight after dialysis was 159.19 g.

Step 2: 114.29 g of the prepared gel were taken and mixed with a second portion of calcium hydroxyapatite, 28.58 g (particle size - 25 - 45 um) for 90 min until homogenization. The homogenized gel was degassed, filled into 1.25 ml syringes and sterilized by steam autoclave at 130 °C for 3 min.

### Test 1: strength of association

The test was performed according to the example 3, at 2040 g-force, using Eppendorf 5415C Centrifuge for 5 min at 5000 rpm. Full separation between the gel and the calcium was observed at gel#3, while only slight difference was observed at gel#4. The results are presented in the table 3 below. Minor deviations from the theoretical 30° calcium hydroxyapatite loading might be attributed to weighing errors.

**Table 3**

| **Gel** | | **Total Gel weight (gr)** | **Dry CaHAp weight (gr)** | **% CaHAp of total gel** | **% CaHAp - Total** |
|---|---|---|---|---|---|
| **Test 1** | Gel #3 (gel) | 1.3610 | 0.0039 | 0.29 | 29.07 |
| | Gel #3 (sediment) | | 0.3917 | 28.78 | |
| **Test 2** | Gel #3 (gel) | 1.3512 | 0.0046 | 0.34 | 27.33 |
| | Gel #3 (sediment) | | 0.3647 | 26.99 | |
| **Test 1** | Gel #4 (gel) | 1.5024 | 0.1804 | 12.01 | 30.83 |
| | Gel #4 (sediment) | | 0.2827 | 18.82 | |
| **Test 2** | Gel #4 (gel) | 1.5422 | 0.1807 | 11.72 | 32.91 |
| | Gel #4 (sediment) | | 0.3268 | 21.19 | |

It can be readily seen that in gel#3 according to the prior art, which contained calcium hydroxyapatite only as loosely associated component, virtually all of the CaHAp (>99%) was separated from the gel. However, at gel#4 at least a one third was retained in the gel. Without being bound by a theory it is believed that CaHAp particles in gel#4 are attached more strongly to the gel and therefore less tend to be separate from it, in a quantity even more that the 10% that were added during the cross-linking stage.

### Test 2: release of calcium hydroxyapatite from the gels

The test was generally performed according to Example 3, with about ~1.5 g of gel used.

The results are presented in the table 4 below. It can be readily seen that even after 6 and 14 days more CaHAp was released from gel#3 than from gel #4.

Without being bound by a theory it is believed that due to the higher association occurring between the particles and the cross-linked gel in gel #4 the release kinetics of the particles is slower. In contrast, gel#3 contained only the particles that were more loosely associated with the gel, which affected their rate of release therefrom.

**Table 4**

| Duration (days) | Gel | Gel weight (gr) | Dry CaHAp weight (gr) | % CaHAp (from the gel) |
|---|---|---|---|---|
| 6 | Gel #3 | 1.5939 | 0.2436 | 15.28 |
| | | 1.5563 | 0.1996 | 12.83 |
| | Gel #4 | 1.5615 | 0.0254 | 1.63 |
| | | 1.5901 | 0.0311 | 1.96 |
| 14 | Gel #3 | 1.5740 | 0.2784 | 17.69 |
| | Gel #4 | 1.5289 | 0.0349 | 2.28 |

### Example 6 - Small-angle X-ray scattering (SAXS)

In addition, some of the gels were tested by Small-angle X-ray scattering (SAXS).

Small angle x-ray scattering (SAXS) patterns of polymer gels were obtained with a SAXSLAB GANESHA 300-XL. CuK_{α} radiation was generated by a Genix 3D Cu-source with an integrated monochromator, 3-pinhole collimation and a two-dimensional Pilatus 300K detector. The scattering intensity I(q) was recorded at intervals of 0.012 < q < 0.6 Å⁻¹. Measurements were performed under vacuum at the ambient temperature.

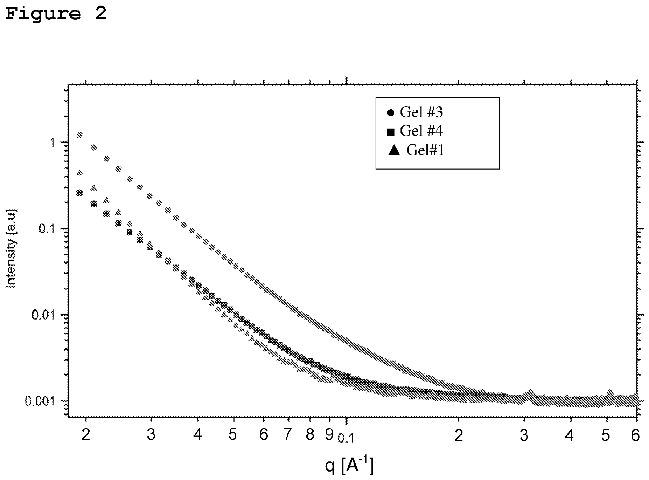

Gels specimens were placed in stainless steel sample cells with entrance and exit windows made of mica. The data analysis was based on fitting the scattering curve by software provided by NIST (NIST SANS analysis version 6.32 on IGOR).] The scattering curves were corrected for counting time and sample absorption. The scattering curves are presented at Figure 2.

Three gels were examined, as described above: gels ## 1, 3 and 4. The SAXS results indicate that the HA matrix in gel in which the calcium hydroxyapatite particles are attached more loosely are more voluminous than the gel comprising also some tightly associated CaHAp particles.

### Example 7 - Low-concentration gels

Further gels comprising low concentration of tightly associated CaHAp and low concentrations of HA were prepared. Additionally, the effect of different Mw of HA was examined.

The gels ##5 and 6 were prepared according to the procedure of the Example 1.

The gels are summarized in the table 5 below, with average EF presented for each gel. It can be readily seen that the tested gels behaved relatively similarly in term of EF values.

**Table 5**

| Gel | Total % CaHAp | CaHAp association | Total % HA in gel | Total % HA in product | Mw of HA | EF (N) |
|---|---|---|---|---|---|---|
| 5 | 18 | 10%-tightly | 1 | 0.87 | ~2 MDa | 12.0 |
| | | 8%-loosely | | | | |
| 6 | 18 | 10%-tightly | 1 | 0.87 | ~3.5 MDa | 12.5 |
| | | 8%-loosely | | | | |

### Example 8 - double-cross-linked gel with tightly associated hydroxyapatite and loosely associated hydroxyapatite

### Step 1: Preparation of a cross-linked gel based on hyaluronic acid.

Sodium hyaluronate (HA) of molecular weight 1.3-2.0 MDa (pharma grade), 2.31 g, was added to 19.19 g water, mixed to dissolution at room temperature, at 300 rpm using a centrifugal mixer, manufactured by Collomix, followed by 0.12 g of 1,4-butanediol diglycidyl ether (BDDE) (supplied by TCI), and the mixture was further mixed for 30 min at 300 rpm. Thereafter, 3.98 g of 1M sodium hydroxide (NaOH) solution were added to the mixture, bringing to a total weight of 25.60 g, at pH >12. The mixture was then placed in an oven set to 45 °C for 3 hours and then placed at 25 °C for additional 15 hours.

### Step 2: Preparation of double cross-linked gel with calcium hydroxyapatite.

A solution of 1M hydrochloric acid (HCl), ca. 2.5-4 mL, was added bringing the mixture to pH ~4 followed by mixing for 15 min. 1M sodium hydroxide (NaOH) ca. 3-5 mL, was added bringing the mixture to pH 12, followed by mixing for 15 min at 300 rpm. Thereafter, 0.11 g 1,4-butanediol diglycidyl ether (BDDE) and 24.00 g of calcium hydroxyapatite Ca₁₀(PO₄)₆(OH)₂ dense microspheres, with an average particle size of 25-45 micrometers (medical grade), were added to the mixture and mixed for 15 min at 300 rpm.

The mixture was then placed in an oven set to 45 °C for 3 hours, and then placed at 25 °C for additional 15 hours.

### Step 3: Preparation of the final bulk.

The mixture was neutralized by adding 80.61 g of neutralization solution to pH 7. The composition of the neutralization solution was 0.25 g of potassium dihydrogen phosphate (KH₂PO₄) (Pharma grade, supplied by Merck), 0.87 g of disodium hydrogen phosphate (Na₂HPO₄) (Pharma grade, supplied by Merck), 76.43 g of water for injection, and 2.83 g of 1M hydrochloric acid (HCl) solution (Pharma grade, supplied by Merck). The mixture was mixed for 1 hour at 300 rpm using the centrifugal mixer, until a homogeneous gel was formed. A second portion of calcium hydroxyapatite, 109.68 g, was added, to the 130.32 g of gel formed after neutralization, and the mixture was further homogenized for 30 min at 300 rpm using the centrifugal mixer. The gel was finally degassed *in vacuo,* by subjecting it to 20 mbar vacuum for 30 minutes, filled into 1.25 mL glass syringes, and sterilized in a steam autoclave at 121 °C for 20 minutes. A cohesive and viscoelastic gel was formed.

The hyaluronic acid concentration in the gel (not including the embedded calcium hydroxyapatite microspheres) is 20 mg/g (2% w/w), its pH is ~7.0 and its osmolality is 300 mOsm/kg. The bulk gel was easily injectable through a needle: a force of 20 to 40 N was required for injecting the gel through a 25G/16mm regular wall needle (manufactured by PIC) at a rate of 1 mL/min.

### Example 9 - double-cross-linked gel with tightly associated hydroxyapatite and loosely associated hydroxyapatite, alternative process

### Step 1: Preparation of a cross-linked gel based on hyaluronic acid with calcium hydroxyapatite.

Sodium hyaluronate (HA) of molecular weight 1.3-2.0 MDa (pharma grade), 2.31 g, was added to 19.19 g water, mixed to dissolution at room temperature, at 300 rpm using a centrifugal mixer, manufactured by Collomix, followed by 0.12 g of 1,4-butanediol diglycidyl ether (BDDE) (supplied by TCI). Thereafter, 3.98 g of 1M sodium hydroxide (NaOH) solution were added to the mixture, bringing to a total weight of 25.60 g, at pH >12. The mixture was mixed for 15 minutes at 300 rpm using the centrifugal mixer. The mixture was then placed in an oven set to 45 °C for 3 hours and then placed at 25 °C for additional 15 hours.

Additional 0.11 g 1,4-butanediol diglycidyl ether (BDDE) and 24.00 g of calcium hydroxyapatite Ca₁₀(PO₄)₆(OH)₂ dense microspheres, with an average particle size of 25-45 micrometers (medical grade), were then added, and mixed for 15 min at 300 rpm using a centrifugal mixer, manufactured by Collomix.

The mixture was then placed in an oven set to 45 °C for 3 hours, and then placed at 25 °C for additional 15 hours.

### Step 2: Preparation of the final bulk.

The mixture was neutralized by adding 80.61 g of neutralization solution to pH 7. The composition of the neutralization solution was 0.25 g of potassium dihydrogen phosphate (KH₂PO₄) (Pharma grade, supplied by Merck), 0.87 g of disodium hydrogen phosphate (Na₂HPO₄) (Pharma grade, supplied by Merck), 76.66 g of water for injection, and 2.83 g of 1M hydrochloric acid (HCl) solution (Pharma grade, supplied by Merck). The mixture was mixed for 1 hour at 300 rpm using the centrifugal mixer, until a homogeneous gel was formed. A second portion of calcium hydroxyapatite, 109.68 g, was added, to the 130.32 g of gel formed after neutralization, and the mixture was further homogenized for 30 min at 300 rpm. The gel was finally degassed *in vacuo,* by subjecting it to 20 mbar vacuum for 30 minutes, filled into 1.25 mL glass syringes, and sterilized in a steam autoclave at 121 °C for 20 minutes. A cohesive and viscoelastic gel was formed.

The hyaluronic acid concentration in the gel (not including the embedded calcium hydroxyapatite microspheres) is 20 mg/g (2% w/w), its pH is ~7.0 and its osmolality is 300 mOsm/kg. The bulk gel was easily injectable through a needle: a force of 20 to 40 N was required for injecting the gel through a 25G/16mm regular wall needle (manufactured by PIC) at a rate of 1 mL/min.

**The following are clauses describing embodiments of the invention. They are not claims.**
1. A process of manufacturing of a gel product comprising cross-linked hyaluronic acid and hydroxyapatite, said process comprising:
   combining in an aqueous medium hyaluronic acid or a salt thereof, a cross-linking agent, and conducting a cross-linking reaction in the presence of a first portion of hydroxyapatite;
   completing the cross-linking reaction; and
   incorporating a second portion of hydroxyapatite into the so-formed gel.
2. The process according to clause 1, wherein said conducting a cross-linking reaction comprises increasing the pH of the medium, and said completing the cross-linking reaction comprises allowing the reaction mixture to stand, and/or neutralizing said reaction mixture.
3. The process of any one of preceding clauses, wherein said first portion of hydroxyapatite is between 5 and 90 weight percent of the total amount of hydroxyapatite.
4. The process of any one of preceding clauses, wherein said first portion of hydroxyapatite is between 5 and 30 weight percent of the total amount of hydroxyapatite.
5. The process of any one of preceding clauses, wherein said cross-linking agent is selected from the group consisting of 1,4-butanediol diglycidyl ether, polyethylene glycol) diglycidyl ether, and ethylene glycol diglycidyl ether.
6. The process of clause 5, wherein said cross-linking agent is 1,4-butanediol diglycidyl ether.
7. The process of any one of preceding, wherein a concentration of said hyaluronic acid in said aqueous medium is between 0.2 and 9 weight percent.
8. The process of clause 7, wherein a concentration of said hyaluronic acid in said aqueous medium is between 0.5-4.
9. The process of any one of preceding, wherein a weight ratio between said hyaluronic acid and said cross-linking agent in said aqueous medium is between 15:1 and 2:1.
10. The process of any one of preceding, further comprising introducing additional amount of hyaluronic acid to the gel after terminating the cross-linking reaction.
11. The process of any one of preceding clauses, wherein a concentration of hydroxyapatite in said gel is above 25 weight percent.
12. The process of clause 11, wherein a concentration of hydroxyapatite in said gel is above 45 weight percent.
13. The process of clause 12, wherein a concentration of hydroxyapatite in said gel is between 50 and 60 weight percent.
14. The process of any one of preceding clauses, wherein said calcium hydroxyapatite has an average particle size between 25 and 45 micrometers.
15. The process of any one of preceding clauses, further comprising at least one of degassing the gel, and/or sterilizing the gel.
16. The process of any one of preceding clauses, comprising charging a reaction vessel with water and hyaluronic acid or a salt thereof, stirring to obtain a solution, adding 1,4-butanediol diglycidyl ether, sodium hydroxide and a first portion of calcium hydroxyapatite, maintaining at elevated temperature for a first period and at ambient temperature for a second period, adding phosphate buffer solution and an aqueous acid to achieve a gel with a nearly neutral pH, and incorporating a second portion of calcium hydroxyapatite into the gel, wherein the weight ratio between the first portion of calcium hydroxyapatite and the second portion of calcium hydroxyapatite is between 1:3 and 1:7, and the total concentration of calcium hydroxyapatite is between 50 and 60 weight percent.
17. An injectable gel composition comprising cross-linked hyaluronic acid and hydroxyapatite, wherein the concentration of said hydroxyapatite is above 45 weight percent of total weight of the gel.
18. The injectable gel of the clause 17, wherein the concentration of said hydroxyapatite is between 50 and 60 weight percent of total weight of the gel.
19. An injectable gel composition comprising cross-linked hyaluronic acid and hydroxyapatite, wherein the concentration of said hydroxyapatite is above 20 weight percent of total weight of the gel, and wherein a portion of said hydroxyapatite is inseparable from said gel following centrifugation for 10 minutes under 735 g-force, said portion being at least about fifth of the total amount of hydroxyapatite.
20. The gel of clause 19, wherein the concentration of said hydroxyapatite is above 25 weight percent of total weight of the gel.
21. The gel of clause 20, wherein the concentration of said hydroxyapatite is above 45 weight percent of total weight of the gel.
22. The gel of clause 21, wherein the concentration of said hydroxyapatite is between 50 and 60 weight percent of total weight of the gel.
23. The gel of any one of clauses 19-22, wherein a concentration of said cross-linked hyaluronic acid is between 0.2 and 9 weight percent.
24. The gel of any one of clauses 19-23, wherein said cross-linked hyaluronic acid is cross-linked with a structural unit corresponding to 1,4-butanediol diglycidyl ether, poly-(ethylene glycol) diglycidyl ether, or ethylene glycol diglycidylether.
25. The gel of any one of clauses 19-24, further comprising non-cross-linked hyaluronic acid.
26. The gel of any one of clauses 19-25, wherein said hydroxyapatite has an average particle size between 25 and 45 micrometers.

## Claims

1. An injectable gel product, said gel product comprising cross-linked hyaluronic acid or a salt thereof, and hydroxyapatite, wherein the concentration of said hydroxyapatite is between 20 and 65 percent by weight of total weight of the gel product, and wherein a portion of between 10% and 60% by weight of said hydroxyapatite in said gel product is tightly-associated hydroxyapatite which is inseparable from said gel product following centrifugation for 10 minutes under 735 g-force.

2. The injectable gel product of claim 1, wherein the concentration of said hydroxyapatite is between 30 and 60 percent by weight of total weight of the gel product.

3. The injectable gel product of any one of claims 1 or 2, wherein the concentration of said hydroxyapatite is between 50 and 60 weight percent of total weight of the gel.

4. The injectable gel product of any one of the preceding claims, wherein a concentration of said cross-linked hyaluronic acid is between 0.2 and 9 weight percent.

5. The injectable gel product of any one of the preceding claims, wherein said cross-linked hyaluronic acid is cross-linked with a structural unit corresponding to 1,4-butanediol diglycidyl ether, poly-(ethylene glycol) diglycidyl ether, or ethylene glycol diglycidylether.

6. The injectable gel product of any one of the preceding claims, further comprising non-cross-linked hyaluronic acid.

7. The injectable gel product of any one of the preceding claims, wherein said hydroxyapatite has an average particle size between 25 and 45 micrometers.

8. The injectable gel product of any one of the preceding claims, wherein said gel product is extrudable through a 25 G/16 mm regular wall needle using a force of about 20 to 40 N at a rate of 1 mL/min.

9. The injectable gel product of any one of the preceding claims, wherein said gel product further comprises up to about 1% lidocaine.

10. The injectable gel product of any one of the preceding claims, wherein the total amount of hydroxyapatite in said gel product is between 55 and 57 weight percent in said gel product.

11. The injectable gel product of any one of the preceding claims, wherein the total amount of hydroxyapatite in said gel product is about 55.7 weight percent in said gel product.

12. The injectable gel product of any one of the preceding claims, wherein the total amount of cross-linked hyaluronic acid is between 0.5 to 1 weight percent in said gel product.
